(19) Europäisches Patentamt
European Patent Office
Office européen des brevets

(11) **EP 3 184 125 B1**

(12) **EUROPÄISCHE PATENTSCHRIFT**

(45) Veröffentlichungstag und Bekanntmachung des Hinweises auf die Patenterteilung:
**18.11.2020   Patentblatt 2020/47**

(51) Int Cl.:
*A61L 26/00* *(2006.01)*        *A61L 15/18* *(2006.01)*
*A61L 15/26* *(2006.01)*        *A61L 15/42* *(2006.01)*

(21) Anmeldenummer: **15201613.5**

(22) Anmeldetag: **21.12.2015**

(54) **HYDROGEL ENTHALTENDES WUNDSYSTEM**

WOUND SYSTEM CONTAINING HYDROGEL

SYSTEME DE SOIN DE BLESSURE CONTENANT DE L'HYDROGEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**28.06.2017   Patentblatt 2017/26**

(73) Patentinhaber: **Paul Hartmann AG**
**89522 Heidenheim (DE)**

(72) Erfinder:
  • **Junginger, Martin**
  **89522 Heidenheim (DE)**
  • **Kettel, Markus**
  **89522 Heidenheim (DE)**

(74) Vertreter: **Ter Meer Steinmeister & Partner Patentanwälte mbB**
**Nymphenburger Straße 4**
**80335 München (DE)**

(56) Entgegenhaltungen:
**WO-A1-2010/000451        WO-A1-2014/178945**
**WO-A2-2010/000450**

**Beschreibung**

Die Erfindung betrifft ein Wundsystem, welches insbesondere in der Wundbehandlung während der Epithelisierungsphase besonders vorteilhaft eingesetzt werden kann. Das Wundsystem umfasst eine Trägerschicht sowie mindestens zwei Hydrogelstreifen, wobei der erste Hydrogelstreifen einen ersten und der zweite Hydrogelstreifen einen zweiten Stoff enthält und wobei der erste und der zweite Stoff unterschiedliche Standardpotentiale aufweisen. Das beschriebene Wundsystem kann insbesondere bei der feuchten Wundbehandlung eingesetzt werden. Darüber hinaus betrifft die Erfindung ein entsprechendes Stoffgemisch aus einem ersten und einem zweiten Stoff, wobei der erste Stoff in einem ersten Hydrogelstreifen und der zweite Stoff in einem zweiten Hydrogelstreifen enthalten sind zur Behandlung von Wunden, bevorzugt während der Epithelisierungsphase.

Die Heilung von Hautwunden beruht auf der Fähigkeit der Haut Epithel- sowie Binde- und Stützgewebe zu regenerieren. Die Regeneration selbst ist durch ein komplexes Geschehen von ineinander übergreifenden Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase).

Unter einer Wunde wird die Trennung des Zusammenhangs von Geweben der Körperhülle bei Menschen oder Tieren verstanden. Sie kann mit einem Verlust an Substanz verbunden sein.

Zur Unterstützung der einzelnen Wundheilungsphasen sind zahlreiche Vorschläge in der Literatur beschrieben. So beschreibt beispielsweise die WO 2010/000451 eine mehrschichtige Wundauflage. Die Wundauflage enthält eine erste Schicht als Wundkontaktschicht, welche bevorzugt eine Hydrogelmatrix, einen Polymerfilm, eine Hydrokolloidmatrix, ein Polymernetz, einen Nonwoven (Vliesstoff) oder ein Adhäsiv umfasst, und eine zweite absorbierende Schicht, die einen hydrophilen Polyurethanschaumstoff mit einem Wassergehalt von mindestens 10 Gew.-% und höchstens 80 Gew.-% Wasser umfasst. Darüber hinaus ist aus der WO 2011/141454 eine Wundauflage zur Wundbehandlung insbesondere im feuchten Milieu mit einem faservliesbasierten Saug-/Spülkörper bekannt, wobei in dem faservliesbasiertem Saug-/Spülkörper superabsorbierendes Material verteilt aufgenommen ist und dieser mit einer salzhaltigen wässrigen Lösung, insbesondere Ringerlösung, vorzugsweise bis zur Sättigung, beaufschlagt ist.

WO2014178945 beschreibt Wundsysteme mit biokompatiblen Elektroden, die schwache elektrische Felder oder schwache Mikroströme generieren.

Der vorliegenden Erfindung liegt die Aufgabe zugrunde, die Wundbehandlung, insbesondere von Wunden in der Epithelisierungsphase, weiter zu verbessern. Es soll mit der vorliegenden Erfindung ein System zur Behandlung von Wunden zur Verfügung gestellt werden, mit dem eine Behandlung möglichst wirksam durchgeführt werden kann und welche beim Patienten nicht als unangenehm empfunden wird. Ferner soll das System vorteilhaft anwendbar sein.

Insbesondere soll die vorliegende Erfindung die Wundheilung in der Epithelisierungs- oder Regenerationsphase vorteilhaft beeinflussen, sodass beispielsweise eine kürzere Behandlungsdauer und/oder geringere Narbenbildung erreicht werden kann.

Die Aufgaben konnten unerwartet durch ein Wundsystem, welches eine Trägerschicht und mindestens zwei Hydrogelstreifen umfasst, gelöst werden, wobei ein erster Hydrogelstreifen einen ersten Stoff und ein zweiter Hydrogelstreifen einen zweiten Stoff mit unterschiedlichem Standardpotential $E°$ enthält, sodass ein niedriger, elektrischer Mikrostrom erzeugt werden kann.

Die Erfindung wird durch die Ansprüche definiert. Gegenstände, die außerhalb des Anspruchsumfanges liegen dienen lediglich zur Information.

Ein Gegenstand der Erfindung ist daher ein Wundsystem, umfassend

(a) Trägerschicht
(b) mindestens zwei Hydrogelstreifen,

wobei ein erster Hydrogelstreifen einen ersten Stoff und ein zweiter Hydrogelstreifen einen zweiten Stoff enthält, und wobei der erste und der zweite Stoff ein unterschiedliches Standardpotential $E°$, gemessen bei 25°C; 101,3 kPa, pH=0, Ionenaktivität = 1, aufweisen, und wobei der erste und/oder der zweite Hydrogelstreifen eine Hydrogelmatrix umfasst, wobei die Hydrogelmatrix ein Polyurethan-Polyharnstoff-Copolymer umfasst, und wobei die Hydrogelstreifen die Wundkontaktschicht sind, und wobei die Hydrogelstreifen der Wunde zugewandt sind und im direkten Kontakt mit der Wunde stehen, und wobei die Trägerschicht einen Polymerschaum umfasst.

Ein weiterer Gegenstand der Erfindung ist ein Stoffgemisch aus einem ersten und zweiten Stoff zur Verwendung in der therapeutischen Behandlung von Wunden am menschlichen oder tierischen Körper, wobei der erste Stoff in einem ersten Hydrogelstreifen und der zweite Stoff in einem zweiten Hydrogelstreifen enthalten sind sowie unterschiedliche Standardpotentiale $E°$ aufweisen, gemessen bei 25 °C; 101,3 kPa, pH=0, Ionenaktivität = 1, wobei die Hydrogelstreifen auf eine Trägerschicht aufgebracht sind und wobei die Trägerschicht einen Polymerschaum umfasst, wobei der erste und/oder der zweite Hydrogelstreifen eine Hydrogelmatrix umfasst und wobei die Hydrogelmatrix ein Polyurethan-Polyharnstoff-Copolymer umfasst, wobei die Hydrogelstreifen die Wundkontaktschicht sind und wobei die Hydrogelstreifen der Wunde zugewandt sind und in direktem Kontakt mit der Wunde stehen, wobei das in den entsprechenden Hydrogelstreifen enthaltene Stoffgemisch zur Verwendung in der therapeutischen Behandlung von Wunden am menschlichen oder tierischen Körper vorzugsweise während der Epithelisierungsphase eingesetzt wird.

Das neue erfindungsgemäße Wundsystem zeichnet sich durch mehrere unerwartete Vorteile aus. Unter anderem kann mittels Befeuchtung, z.B. durch die Wundflüssigkeit, ein geringfügiger elektrischer Stromfluss erzeugt werden, der insbesondere während der Epithelisierungsphase zu unerwartet vorteilhaften Wundheilung und/oder die vorteilhaften Verminderung der Narbenbildung führt.

Die Bestandteile (a) und (b) des erfindungsgemäßen Wundsystems werden nachstehend beschrieben.

Das erfindungsgemäße Wundsystem umfasst eine Trägerschicht (a) die einen Polymerschaum umfasst.

Diese Trägerschicht kann bevorzugt einen Film, einen Schaum, ein Gewebe, ein Gestrick, ein Filament, einen Vliesstoff (auch als "Nonwoven" bezeichnet), ein Netz, verwobene Materialien und/oder Kombinationen daraus umfassen.

Die Trägerschicht kann aus verschiedenen Materialien bestehen. In einer bevorzugten Ausführungsform kann die Trägerschicht (a) ein natürliches Material enthalten, wobei ein natürliches Material bevorzugt pflanzlicher oder tierischer Herkunft ist. Beispiele für natürliche Materialien sind natürliche Schwämme, Polypeptide, Pektine, Seiden, Keratine, Alginate und natürliche Fasern wie Baumwolle, Seide oder Zellstoff sowie Gemische davon.

Alternativ bevorzugt kann die Trägerschicht ein synthetisches Material enthalten. Synthetische Materialien können auch natürlichen Ursprungs sein, wobei sie durch anschließende Behandlung, wie eine chemische Umsetzung, verändert werden. Beispiele sind Polymere wie Polyurethane, Polyolefine, Polyvinylalkohol, Poly(meth)acrylate, Acrylate, Kunstseide, Elastomere, künstliche Cellulosen wie Methylcellulose, Ethylcellulose und Hydroxypropylmethylcellulose und künstliche Schwämme und Schäume sowie Gemische davon.

In einer alternativ bevorzugten Ausführungsform ist die Trägerschicht (a) wasserbeziehungsweise flüssigkeitsdurchlässig. Unter wasser- beziehungsweise flüssigkeitsdurchlässig soll verstanden werden, dass die Schicht einen Wasser- beziehungsweise Flüssigkeitsdurchgang von der einen Seite der ersten Schicht zur anderen Seite der ersten Schicht nicht vollständig unterbindet. In einer bevorzugten Ausführungsform kann die erste Schicht eine Vielzahl von Kanälen zum Durchtritt von Flüssigkeit umfassen.

In einer bevorzugten Ausführungsform ist die Trägerschicht (a) wasser- beziehungsweise flüssigkeitsundurchlässig. Unter wasser- beziehungsweise flüssigkeitsundurchlässig soll verstanden werden, dass die Schicht einen Flüssigkeitsdurchgang von der einen Seite der ersten Schicht zur anderen Seite der ersten Schicht vollständig unterbindet.

In der vorliegenden Erfindung umfasst die Trägerschicht (a) einen Polymerschaum. Bevorzugt sind Polymerschäume, die wasser- beziehungsweise flüssigkeitsundurchlässig sind. Hier sind besonders Schäume geeignet, die aus Polyurethan, Polyetherurethan, Polyesterurethan, Polyether-Polyamid-Copolymeren, Polyacrylat und Polymethacrylat gefertigt werden. Sehr bevorzugt ist als Trägerschicht ein wasserundurchlässiger Polyurethanschaum.

Die Trägerschicht kann direkten oder indirekten Kontakt zu den mindestens zwei Hydrogelstreifen (b) haben. Bei einem direkten Kontakt werden die mindestens zwei Hydrogelstreifen beispielsweise direkt auf die Trägerschicht gedruckt. Bei indirektem Kontakt werden die mindestens zwei Hydrogelstreifen beispielsweise mittels eines Adhäsivs auf die Trägerschicht aufgebracht.

Das erfindungsgemäße Wundsystem umfasst mindestens zwei Hydrogelstreifen, wobei ein erster Hydrogelstreifen einen ersten Stoff und ein zweiter Hydrogelstreifen einen zweiten Stoff enthält, wobei der erste und der zweite Stoff ein unterschiedliches Standardpotential $E°$ aufweisen, gemessen bei 25 °C; 101,3 kPa, pH=0, Ionenaktivität = 1.

Das Hydrogel umfasst gemäß der vorliegenden Erfindung eine Hydrogelmatrix.

Gemäß der vorliegenden Erfindung umfasst die Hydrogelmatrix ein Polyurethan-Polyharnstoff-Copolymer. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelmatrix ist besonders gut geeignet Wasser einzulagern und dieses Wasser an eine Wunde abzugeben.

Weiterhin bevorzugt kann die wasserhaltige Hydrogelmatrix weiterhin mindestens einen mehrwertigen Alkohol aus der Gruppe der zweiwertigen, dreiwertigen, vierwertigen, fünfwertigen oder sechswertigen Alkohole umfassen. Insbesondere kann der Alkohol gewählt werden aus der Gruppe der Glykole, insbesondere Ethylenglykol oder Propylenglykol, sowie Sorbitol oder Glycerin oder Mischungen hiervon. Diese Alkohole sind hervorragend als Feuchtigkeitsspender geeignet und stellen somit für die die Wunde umgebende Haut eine pflegende Komponente dar.

Dabei kann die wasserhaltige Hydrogelmatrix insbesondere 0 bis 50 Gew.-% eines mehrwertigen Alkohols umfassen. Insbesondere umfasst die Hydrogelmatrix 5 bis 40 Gew.- % eines mehrwertigen Alkohols und ganz besonders bevorzugt 10 bis 30 Gew.-% eines mehrwertigen Alkohols.

Im feuchten Zustand kann die Hydrogelmatrix vorzugsweise mindestens 20 Gew.-%, mehr bevorzugt mindestens 30 Gew.-%, noch mehr bevorzugt mindestens 40 Gew.-%, insbesondere mindestens 50 Gew.-% Wasser umfassen, wobei die Hydrogelmatrix weiterhin bevorzugt höchstens 90 Gew.-% und weiterhin bevorzugt höchstens 80 Gew.-% Wasser umfasst. Somit kann ein Wundsystem bereitgestellt werden, das eine wasserhaltige Komponente enthält und somit eine für die natürliche Heilung ausreichende Menge an Wasser zur Verfügung stellt.

Zur Überprüfung der Menge an Wasser einer Komponente soll im Zusammenhang der vorliegenden Erfindung die Norm DIN EN 14079 herangezogen werden, wobei die Menge an Wasser wie folgt berechnet wird:

$$W_w = \frac{W_g - W_t}{W_g} \bullet 100\% \qquad (1)$$

mit

$W_w$ = Gewicht des Wassers in %, bezogen auf das Gesamtgewicht der Komponente
$W_g$ = Gewicht der Wasser enthaltenden Komponente
$W_t$ = Gewicht der trockenen Komponente.

Im Zusammenhang mit der vorliegenden Erfindung soll unter einem Wasseranteil das Wasser verstanden werden, welches theoretisch aus dem Hydrogel freigesetzt werden kann. Im Unterschied hierzu ist nicht der Anteil an Wasser zu verstehen, der eventuell zur Bildung, beispielsweise bei der Polymerisation der Ausgangsprodukte des Polyurethanschaums, verwendet wird. Dieses Wasser wird kovalent gebunden und steht nicht der Wundbehandlung zur Verfügung. Darüber hinaus soll auch nicht ein Wasseranteil verstanden sein, der produktionsbedingt bei einer möglichen Herstellung des Hydrogels verwendet wird. Dieser Wasseranteil kann nach oder während der Bildung des Hydrogels beziehungsweise der Hydrogelmatrix meist durch Trocknen, beispielsweise durch Trocknen in einem Ofen, entzogen und steht somit auch nicht der Wundbehandlung zur Verfügung.

Darüber hinaus kann vorgesehen sein, dass der erste und/oder zweite Hydrogelstreifen (neben dem ersten Stoff und den zweiten Stoff) zusätzlich ein Salz umfasst. Insbesondere ist hierbei vorgesehen, dass es sich um ein anorganisches Salz handelt. Besonders bevorzugt sind in diesem Zusammenhang Salze, bei denen das Kation ein Alkali- oder Erdalkalimetall oder Mischungen davon, vorzugsweise Natrium, Kalium, Magnesium oder Kalzium oder Mischungen davon, umfasst. Weiterhin ist das Anion bevorzugt ein Sulfat, Hydrogensulfat, Carbonat, Hydrogencarbonat, Phosphat, Hydrogenphosphat, Halogenid, besonders bevorzugt ein Chlorid oder Iodid, oder Mischungen davon. Ganz besonders bevorzugt umfasst der erste und/oder zweite Hydrogelstreifen Natriumchlorid, Kaliumchlorid, Magnesiumchlorid, Kalziumchlorid oder Mischungen hiervon. Diese Salze simulieren in besonders guter Weise das Elektrolyt-Gemisch in einem von einer Wunde abgegebenen Wundserum. Damit stellt eine diese Salze umfassende Hydrogelmatrix einer Wunde ein besonders wundheilungsförderndes Klima zur Verfügung. Hierbei kann vorgesehen sein, dass die Hydrogelmatrix 0 bis 5 Gew.-% mindestens eines Salzes umfasst. Insbesondere umfasst die Hydrogelmatrix 0,1 bis 3 Gew.-% eines Salzes und ganz besonders bevorzugt 0,5 bis 1,5 Gew.-% eines Salzes.

Insgesamt kann gemäß der vorliegenden Erfindung vorgesehen sein, dass die wasserhaltige Hydrogelmatrix bevorzugt mindestens 20 Gew.-% Wasser und mindestens 10 Gew.-% Polyurethan-Polyharnstoff-Copolymer umfasst. Eine weiterhin bevorzugte Hydrogelmatrix umfasst mindestens 20 Gew.-% Wasser und mindestens 15 Gew.-% Polyurethan-Polyharnstoff-Copolymer. Hierbei kann weiterhin bevorzugt vorgesehen sein, dass die Hydrogelmatrix aus 6 bis 60 Gew.-% eines Präpolymers mit aliphatischen Diisocyanat-Gruppen, 4 bis 40 Gew.-% Polyamin auf Polyethylenoxidbasis, 0 bis 50 Gew.-% eines mehrwertigen Alkohols, 0 bis 5 Gew.-% mindestens eines Salzes ausgewählt aus der Gruppe Natriumchlorid, Kaliumchlorid, Magnesiumchlorid, Kalziumchlorid oder Mischungen hiervon und mindestens 20 Gew.-% Wasser gebildet wird.

Weiterhin bevorzugt kann vorgesehen sein, dass die Hydrogelmatrix aus 6 bis 30 Gew.-% eines Präpolymers mit aliphatischen Diisocyanat-Gruppen, 4 bis 20 Gew.-% Diamin auf Polyethylenoxidbasis, 10 bis 30 Gew.-% eines mehrwertigen Alkohols ausgewählt aus der Gruppe bestehend aus Propylenglycol und/oder Glycerin, 0,5 - 1,5 Gew.-% eines Salzes ausgewählt aus der Gruppe Natriumchlorid, Kaliumchlorid, Magnesiumchlorid, Kalziumchlorid oder Mischungen hiervon und mindestens 30 Gew.-% Wasser gebildet wird.

Ganz besonders bevorzugt kann vorgesehen sein, dass die Hydrogelmatrix aus 6 bis 20 Gew.-% Präpolymer mit Isophorondiisocyanat-Enden, 4 bis 15 Gew.-% Diamin auf Polyethylenoxidbasis, 15 bis 20 Gew.-% Polypropylenglycol und/oder Glycerin, 0,5 bis 1,5 Gew.-% eines Salzes ausgewählt aus der Gruppe Natriumchlorid, Kaliumchlorid, Magnesiumchlorid, Kalziumchlorid oder Mischungen hiervon und mindestens 40 Gew.-% Wasser gebildet wird. Diese Hydrogelmatrix weist eine freie Absorption A3 (gemessen nach DIN EN 13723-1 (2002)) von mindestens 1 g/g und höchstens 5 g/g auf, stellt ein nicht reizendes, flüssigkeitsabsorbierendes, vor Bakterien schützendes, polsterndes, hautartiges Medium bereit.

In einer bevorzugten Ausführungsform der Erfindung weist die Hydrogelmatrix eine Schichtdicke 0,1 bis 5,0 mm, insbesondere von 0,5 bis 5,0 mm und ganz besonders bevorzugt von 0,5 bis 3,0 mm auf.

Es ist bevorzugt, dass der erste und der zweite Hydrogelstreifen das gleiche Hydrogel oder die gleiche Hydrogelmatrix aufweisen. Alternativ können der erste und zweite Hydrogelstreifen auch ein unterschiedliches Hydrogel oder eine unterschiedliche Hydrogelmatrix aufweisen.

Das Wundsystem der vorliegenden Erfindung umfasst mindestens zwei Hydrogelstreifen, wobei ein erster Hydrogelstreifen einen ersten Stoff enthält. In einer bevorzugten Ausführungsform kann das vorliegende Wundsystem mehrere erste Hydrogelstreifen, die einen ersten Stoff enthalten, umfassen. Der erste Stoff ist in dem ersten Hydrogelstreifen enthalten. In einer bevorzugten Ausführungsform ist der erste Stoff in dem ersten Hydrogelstreifen verteilt, vorzugsweise homogen oder statistisch verteilt. In einer alternativen Form kann der erste Stoff auch in Form eines Drahts in dem ersten Hydrogelstreifen enthalten sein.

Das Wundsystem der vorliegenden Erfindung umfasst mindestens zwei Hydrogelstreifen, wobei ein zweiter Hydrogelstreifen einen zweiten Stoff enthält. In einer bevorzugten Ausführungsform kann das vorliegende Wundsystem mehrere zweite Hydrogelstreifen, die einen zweiten Stoff enthalten, umfassen Der zweite Stoff ist in dem zweiten Hydrogelstreifen enthalten. In einer bevorzugten Ausführungsform ist der zweite Stoff in dem zweiten Hydrogelstreifen verteilt, vorzugsweise homogen oder statistisch verteilt. In einer alternativen Form kann der zweite Stoff auch in Form eines Drahts in den zweiten Hydrogelstreifen enthalten sein.

Sowohl der/die den ersten Stoff enthaltende(n) ersten Hydrogelstreifen als auch der/die den zweiten Stoff enthaltende(n) Hydrogelstreifen liegen vorzugsweise in Form eines mathematischen Zylinders vor. Ein mathematischer Zylinder kann wie folgt definiert werden: Eine ebene Kurve $c_0$ in einer Ebene $\varepsilon_0$ wird entlang einer Gerade, die nicht in $\varepsilon_0$ enthalten ist, um eine feste Strecke $\vec{a}$ verschoben. Je zwei sich entsprechende Punkte der Kurven $c_0$ und der verschobenen Kurve $c_1$ werden durch eine Strecke verbunden. Die Gesamtheit dieser parallelen Strecken bildet die zugehörige Zylinderfläche. Die Kurve $c_0$ nennt man Leitkurve. Ist die ebene Kurve ein Kreis, entsteht ein schiefer Kreiszylinder. Falls $\vec{a} \perp \varepsilon_0$ ist, ergibt sich ein senkrechter Kreiszylinder.

Ist $c_0$ eine geschlossene Kurve, kann man die Mantelfläche mit den beiden Begrenzungsflächen wieder als Oberfläche eines Körpers auffassen. Ist die Kurve $c_0$ nicht geschlossen, z.B. ein Parabelbogen (siehe unten), so ist der Zylinder nur die oben erklärte Mantelfläche, die allerdings Teil einer Oberfläche eines Körpers sein kann.

Als besonders vorteilhafte Ausführungsform hat sich gezeigt, dass die ersten und zweiten Hydrogelstreifen eine Dicke von 0,1 bis 10 mm, insbesondere von 0,5 bis 5 mm und ganz besonders bevorzugt von 0,5 bis 3 mm aufweisen. In einer bevorzugten Ausführungsform hat der erste und/oder der zweite Hydrogelstreifen eine Länge von 1 bis 100 mm, bevorzugt von 5 bis 75 mm, insbesondere von 10 bis 50 mm. Solche Abmaße weisen einerseits die Fähigkeit auf, ein von einer Wunde abgegebenes Wundexsudat aufzunehmen und können gleichzeitig einer Wunde eine ausreichende Menge an Wasser oder Feuchtigkeit sowie eine gute Verfügbarkeit der ersten und zweiten Stoffe bereitstellen. Diese Abmaße können an jeder Stelle gleich sein oder in verschiedenen Bereichen verschiedene Werte annehmen.

Im erfindungsgemäßen Wundsystem enthält der erste Hydrogelstreifen einen ersten Stoff und der zweite Hydrogelstreifen enthält einen zweiten Stoff. In einer Ausführungsform ist der erste Stoff in dem Hydrogelstreifen vorzugsweise vollständig enthalten; das bedeutet, dass der erste Stoff vollständig innerhalb des ersten Hydrogelstreifens und nicht an dessen Oberfläche vorliegt. Der zweite Stoff liegt ebenfalls vorzugsweise vollständig im zweiten Hydrogelstreifen vor. Somit stehen der erste und der zweite Stoff vorzugsweise nicht in direktem Kontakt miteinander.

In einer bevorzugten Ausführungsform können der erste Stoff, der in dem ersten Hydrogelstreifen enthalten ist, und der zweite Stoff, der in dem zweiten Hydrogelstreifen enthalten ist, und welche ein unterschiedliches Standardpotential $E°$ aufweisen, als Elektroden betrachtet werden. Durch das unterschiedliche Standardpotential $E°$ der ersten und zweiten Substanz kann eine Spannung zwischen den Elektroden vorliegen. Gegebenenfalls kann auch ein elektrischer Mikrostrom fließen sobald die Elektroden durch einen Elektrolyten verbunden werden.

In einer bevorzugten Ausführungsform beträgt der elektrische Mikrostrom 1 μA bis 800 μA, bevorzugt 10 μA bis 600 μA, mehr bevorzugt 25 μA bis 400 μA, insbesondere 50 μA bis 200 μA.

In einer bevorzugten Ausführungsform umfasst das erfindungsgemäße Wundsystem mehrere einen ersten Stoff enthaltende erste Hydrogelstreifen sowie mehrere einen zweiten Stoff enthaltende zweite Hydrogelstreifen.

Durch die unterschiedliche Anordnung der ersten den ersten Stoff enthaltenden Hydrogelstreifen und der zweiten den zweiten Stoff enthaltenden Hydrogelstreifen können unterschiedliche Muster (Pattern) gebildet werden, nach denen ein Stromfluss möglich ist.

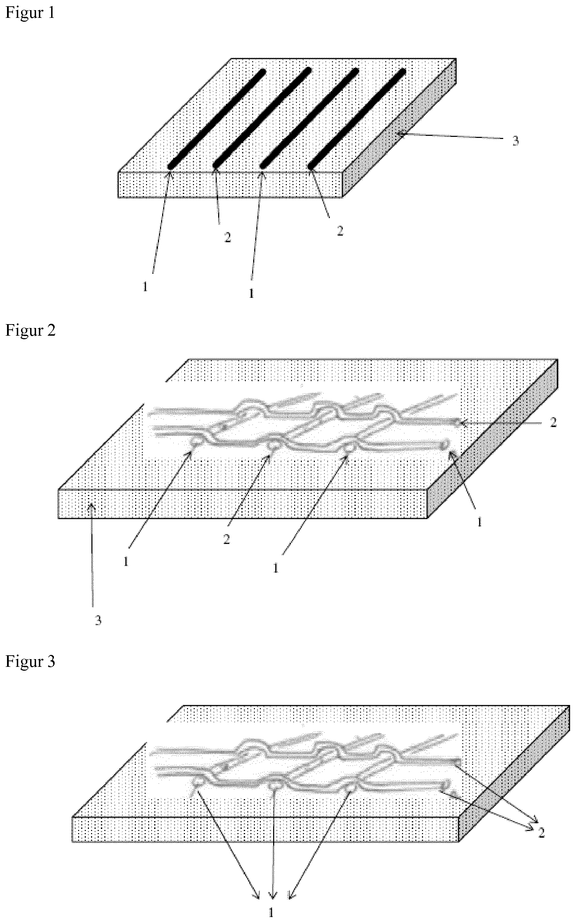

In einer bevorzugten Ausführungsform liegen abwechselnd erste und zweite Hydrogelstreifen auf der Trägerschicht vor. Eine entsprechende Ausführungsform kann durch Figur 1 veranschaulicht werden.

In Figur 1 bedeuten

1 erster Hydrogelstreifen, welcher einen ersten Stoff enthält,
2 zweiter Hydrogelstreifen, welcher einen zweiten Stoff enthält

3 Trägerschicht

In einer alternativ bevorzugten Ausführungsform liegen abwechselnd erste und zweite Hydrogelstreifen auf der Trägerschicht vor, wobei oberhalb der auf der Trägerschicht liegenden ersten und zweiten Hydrogelstreifen wiederum abwechselnd weitere erste und zweite Hydrogelstreifen aufgebracht sind. In diesem Fall sieht die Anordnung wie ein Netzwerk oder Gitter aus, wobei erste und zweite Hydrogelstreifen immer abwechselnd angeordnet sind. Eine entsprechende Ausführungsform kann durch Figur 2 veranschaulicht werden.

In Figur 2 bedeuten

1 erster Hydrogelstreifen, welcher einen ersten Stoff enthält,
2 zweiter Hydrogelstreifen, welcher einen zweiten Stoff enthält
3 Trägerschicht

In einer weiteren alternativ bevorzugten Ausführungsform liegen auf der Trägerschicht nebeneinander erste Hydrogelstreifen vor und oberhalb der auf der Trägerschicht liegenden ersten Hydrogelstreifen sind nebeneinander liegend zweite Hydrogelstreifen, vorzugsweise in einem Winkel dazu, bevorzugt etwa 90°, angebracht. In diesem Fall sieht die Anordnung wie ein Netzwerk oder Gitter aus, wobei in einer Ebene erste und in der nächsten Ebene zweite Hydrogelstreifen angeordnet sind. Alternativ bevorzugt ist auch die umgekehrte Anordnung; d.h. auf der Trägerschicht liegen nebeneinander angeordnet zweite Hydrogelstreifen und auf diesen sind dann die ersten Hydrogelstreifen angeordnet.

Die mindestens zwei Hydrogelstreifen werden bevorzugt mit Hilfe eines Druckverfahrens direkt oder indirekt mithilfe eines Adhäsivs auf die Trägerschicht aufgebracht. Bevorzugt ist die Aufbringung direkt auf die Trägerschicht. Eine entsprechende Ausführungsform kann durch Figur 3 veranschaulicht werden. In Figur 3 bedeuten

1 erster Hydrogelstreifen, welcher einen ersten Stoff enthält,
2 zweiter Hydrogelstreifen, welcher einen zweiten Stoff enthält
3 Trägerschicht

Weiterhin ist es (in allen Ausführungsformen) bevorzugt, dass die Hydrogelstreifen beziehungsweise die Hydrogelmatrix erst direkt vor der Anwendung mit Feuchtigkeit, bevorzugt mit Wasser oder mit einer salzhaltigen Lösung, bevorzugt mit einer salzhaltigen Lösung, getränkt werden.

Bei der salzhaltigen Lösung handelt es sich bevorzugt um eine salzhaltige wässrige Lösung. Geeignete Salze zum Fertigen der Lösung sind alle physiologisch verträglichen Salze. Geeignete Salze sind beispielsweise Natriumchlorid NaCl, Kaliumchlorid KCl, Kaliumiodid KI, Kaliumsulfat $K_2SO_4$, Magnesiumsulfat $MgSO_4$, Calciumchlorid $CaCl_2$, Eisenphosphat $FePO_4$ und Gemische davon. Bevorzugt handelt es sich bei der wässrigen Salzlösung um eine Ringerlösung, welche typischerweise Natriumchlorid, Kaliumchlorid und Calciumchlorid, insbesondere 8,6 g NaCl, 0,3 g KCl und 0,33 g $CaCl_2$ je Liter enthält.

Bevorzugt kann die wässrige, salzhaltige Lösung eine bei pH-Werten von 4 bis 7,5 eines typischen feuchten oder feuchtnassen Wundmilieus kationische Substanz mit antimikrobieller Wirkung umfassen. Diese Substanz kann bevorzugt von negativen Gruppen des Hydrogels attraktiv angezogen werden und so innerhalb des Hydrogelstreifens antimikrobiell wirken. Somit kann eine hervorragende Konditionierung des Hydrogelstreifens auch für Anwendungen über 24 Stunden hinaus realisiert werden, was sich wiederum vorteilhaft auf die Konditionierung des Wundmilieus auswirkt, da im Flüssigkeitsaustausch im Betrieb des Hydrogels Flüssigkeit aus dem antimikrobiell optimal konditionierten Hydrogels zur Wunde gelangt, jedoch im Wesentlichen ohne dass hierbei septisch wirkende Substanzen oder Keime zurück zur Wunde gelangen. Es wird also die Rückkontamination weitestgehend verhindert.

Bei der kationischen Substanz mit antimikrobieller Wirkung kann es sich beispielsweise um Substanzen mit Amino- oder Iminogruppen handeln, welche in einer Lösung mit einem pH-Wert von 4 bis 7,5 kationisch geladen vorliegen. Besonders geeignete kationische Substanzen mit antimikrobieller Wirkung sind Biguanid-Derivate wie Chlorhexidin oder Polybiguanide, wie Polyethylene Biguanid (PEB), Polytetramethylenbiguanid (PTMB) oder Polyethylenhexamethylenbiguanide (PEHMB). Ein besonders bevorzugtes Polybiguanid ist Polyhexamethylenbiguanid (PHMB, bzw. Polyhexanid). Weitere geeignete kationische Substanzen mit antimikrobieller Wirkung sind Polyguanidine wie zum Beispiel Polyhexamethylenbiguanid (PHMG), *N*-Octyl-1-[10-(4-octyliminopyridin-1-yl)decyl]pyridin-4-imin (Octenidin), quartäre Ammonium-verbindungen, wie zum Beispiel Benzalkoniumchlorid oder Cetylpyridiniumchlorid, Triazine wie zum Beispiel 1-(3-Chloroallyl)-3,5,7-triaza-1-azoniaadamantan-chlorid oder die Ammoniumverbindung Taurolidin.

Die Konzentration des antiseptisch wirkenden Zusatzes in der wässrigen Lösung, die dann zur Aktivierung der trockenen Hydrogelmatrix verwendet wird, beträgt vorzugsweise 0,06 bis 0,20 Gew.-%, insbesondere 0,08 bis 0,15 Gew.-%, insbesondere 0,10 bis 0,15 Gew.-% (bezogen auf die wässrige Lösung vor dem Aktivieren des Hydrogels beziehungsweise der Hydrogelmatrix).

Wie oben bereits ausgeführt, wird durch die Wahl der Substanz mit antimikrobieller Wirkung im Zusammenwirken mit dem Hydrogel erreicht, dass diese Substanz auch im Betrieb der Wundsystems weitgehend oder weitestgehend innerhalb der Hydrogelstreifen verbleibt. Dies kann anhand des Hemmhoftests (Englisch: zone of inhibition test) visuell geprüft bzw. verifiziert werden. Bei der Durchführung des nachfolgend im Einzelnen beschriebenen Hemmhoftests kann visuell überprüft werden, ob aus einem Testkörper keimtötende Substanzen in wesentlicher Menge austreten oder nicht. Treten sie aus, so töten oder behindern sie das Wachstum sogenannter koloniebildender Einheiten in einer Umgebung des Testkörpers, was visuell durch Inaugenscheinnahme des Testkörpers und seines Umfelds festgestellt werden kann. Lässt sich keine solche Umgebung, also kein Hemmhof (oder englisch: zone of inhibition) feststellen, so bedeutet dies, dass aus dem Testkörper keine das Koloniewachstum in nennenswertem Umfang beeinträchtigende Substanzen ausgetreten sind. Zur noch weitergehenden Charakterisierung des Gegenstands der vorliegenden Erfindung ist die Wundauflage weiter dadurch gekennzeichnet, dass bei Ausführung des Hemmhoftests ein Austreten von kationischer Substanz mit antimikrobieller Wirkung aus der Wundauflage visuell nicht feststellbar ist. Dies bedeutet, dass ein Hemmhof bei Ausführung des nachfolgend beschriebenen Tests visuell nicht feststellbar ist.

Zur Durchführung des Hemmhoftests wird ein Wundsystem der hier in Rede stehenden Art als Testkörper auf eine zuvor vorbereitete Agarplatte aufgelegt. Es wurde hierfür eine Agarplatte mit einem Durchmesser von 8,5 cm eingesetzt, die jeweils 15 ml Caseinpepton-Sojapepton-Agar enthielt (Konzentration des Caseinpepton-Sojapepton: 40 g/l). Auf diese Agarplatte wurden 100 $\mu$l einer Staphylokokkus aureus ATCC 6538 Keimsuspension (etwa 5 x 10$^6$ Koloniebildende Einheiten/ml) mit einem Wattestäbchen aufgestrichen und für 10 bis 15 Minuten getrocknet. Nach dem Trocknen der Keimsuspension bei geschlossenem Deckel wird der sterilisierte, jedoch auf Raumtemperatur abgekühlte, Testkörper in Form der zu testenden Wundauflage auf die Agarplatte mit der wundzugewandten Seite aufgelegt. Die Platte wird dann aufrecht stehend für 18 Stunden bei 35°C inkubiert. Danach wird die Agarschale visuell nach der Bildung einer Hemmzone untersucht. Die Hemmzone wird typischerweise in Millimeter nach der Formel H = (D-d) : 2 berechnet, wobei D der Gesamtdurchmesser von Testkörper und Hemmzone und d der Durchmesser des Testkörpers, jeweils in Millimeter, ist. Sofern eine Hemmzone um den Testkörper herum visuell nicht feststellbar ist (D = d und H = 0), so ist der Austritt etwaiger Substanzen aus dem Testkörper und deren Auswirkung auf das Koloniewachstum nach dem Hemmhoftest nicht feststellbar.

Durch das Tränken der Hydrogelstreifen beziehungsweise der Hydrogelmatrix/ces erst direkt vor der Verwendung in der Wundbehandlung mit Feuchtigkeit, bevorzugt mit Wasser oder mit einer salzhaltigen Lösung, kann verhindert werden, dass sich bereits während der Lagerung eine Elektrolytlösung zwischen dem ersten und zweiten Stoff befindet. Damit wird die vorzeitige Bildung eines elektrischen Mikrostroms verhindert, vorzugsweise vollständig verhindert.

In einer bevorzugten Ausführungsform sind der erste und der zweite Stoff jeweils ein Metall und/oder ein entsprechendes Metallsalz.

Beispiele für geeignete Metalle sind Gold (Au), Platin (Pt), Silber (Ag), Kupfer (Cu), Eisen (Fe), Zinn (Sn), Zink (Zn), Mangan (Mn), Titan (Ti), Aluminium (Al), Magnesium (Mg) und Legierungen daraus.

Ein Metallsalz der oben aufgeführten beispielsweisen Metalle umfasst neben dem entsprechenden Metallkation ein oder mehrere Anionen. Beispiele für Anionen sind Oxide, Sulfide, Sulfate, Nitrate, Halogenide wie Fluorid, Chlorid, Bromid und Iodid sowie Gemische daraus.

In einer bevorzugten Ausführungsform ist der erste Stoff Zink. Zink ist ein Metall, welches ein Standardpotential $E°$ von -0,76 Volt aufweist. Zink wird gewöhnlich als Reduktionsmittel eingesetzt, wobei Zink unter Elektronenabgabe zu $Zn^{2+}$ oxidiert wird. Zink zählt zu den essentiellen Spurenelementen für den menschlichen und tierischen Körper.

In einer bevorzugten Ausführungsform ist der zweite Stoff Silber und/oder ein Silbersalz. Beispiele für geeignete Silbersalze sind Silberhalogenide, wie Silberfluorid, Silberchlorid, Silberbromid, Silberiodid, Silberoxid und Gemische davon. Es ist besonders bevorzugt, dass der zweite Stoff eine Kombination aus Silber und dem entsprechenden Silbersalz ist. Silber ist ein Metall, welches ein Standardpotential $E°$ von 0,80 Volt aufweist. Für gewöhnlich werden Silberkationen als Oxidationsmittel eingesetzt, wobei sie unter Elektronenaufnahme zu Silber reduziert werden. Silber wirkt bakterizid und kann aus diesem Grund vorteilhaft im Rahmen einer Wundbehandlung/Wundheilung eingesetzt werden. Silberionen können ebenfalls in der Wundtherapie eingesetzt werden und wirken zudem desinfizierend. Weiterhin können Silber und Silberionen durch Hemmung des Bakterienwachstums unangenehme Gerüche verhindern.

In eine alternativ bevorzugten Ausführungsform ist der erste Stoff Silber und/oder ein Silbersalz und der zweite Stoff Zink.

Gemäß der Erfindung sind die Hydrogelstreifen die Wundkontaktschicht, d.h. das Wundsystem ist so angeordnet, dass die Hydrogelstreifen der Wunde zugewandt sind und in direktem Kontakt mit der Wunde stehen Die Wunde sowie der erste und der zweite Stoff werden von Anfang an befeuchtet. Hierdurch werden die Wundheilung eventuell negativ beeinflussende Faktoren der Wundoberfläche entzogen und gleichzeitig Feuchtigkeit und Wasser in einer ausreichenden Menge zur Verfügung gestellt.

Weiterhin erweist es sich als wesentlich, dass hinreichend Flüssigkeit in dem Wundsystem über die gesamte Dauer der Verwendung, also vorzugsweise bis zum nächsten Verbandwechsel, zur Verfügung steht, damit die eingangs genannte duale Funktion des Wundsystems auch tatsächlich wirksam wird.

In einer bevorzugten Ausführungsform kann das Wundsystem in der Epithelisierungsphase der Wundheilung eingesetzt werde. Es hat sich heraus gestellt, dass das Feuchthalten der Wunde in Kombination mit der Anwendung eines geringen elektrischen Stroms eine vorteilhafte Auswirkung auf den Heilungsprozess hat. So kann dieser bevorzugt verkürzt werden und/oder die Narbenbildung kann vorteilhaft reduziert werden.

Ein weiterer Gegenstand der Erfindung ist ein erster und zweiter Stoff zur Verwendung in der therapeutischen Behandlung von Wunden am menschlichen und tierischen Körper, wobei der erste Stoff in einem ersten Hydrogelstreifen und der zweite Stoff in einem zweiten Hydrogelstreifen enthalten sind, wobei die Hydrogelstreifen auf eine Trägerschicht aufgebracht sind, und wobei der erste und der zweite Stoff ein unterschiedliches Standardpotential $E°$ aufweisen, gemessen bei 25°C; 101,3 kPa, pH=0; Ionenaktivität = 1, und wobei der erste und/oder der zweite Hydrogelstreifen eine Hydrogelmatrix umfasst, wobei die Hydrogelmatrix ein Polyurethan-Polyharnstoff-Copolymer umfasst, und wobei die Hydrogelstreifen die Wundkontaktschicht sind, und wobei die Hydrogelstreifen der Wunde zugewandt sind und im direkten Kontakt mit der Wunde stehen, und wobei die Trägerschicht einen Polymerschaum umfasst. Der in dem ersten Hydrogelstreifen enthaltene erste Stoff und der in dem zweiten Hydrogelstreifen enthaltene zweite Stoff können somit ein Stoffgemisch darstellen, welches durch chemische und physikalische Interaktion mit menschlichen Körperzellen die Wundheilung positiv beeinflusst.

Erster und zweiter Stoff sowie erster und zweiter Hydrogelstreifen weisen die oben beschriebenen Eigenschaften auf. Das heißt, die Erläuterungen zu bevorzugten Ausführungsformen für das erfindungsgemäße System finden auch für die erfindungsgemäßen Stoffe bzw. Schichten Anwendung.

In einer bevorzugten Ausführungsform werden der erste in dem ersten Hydrogelstreifen enthaltene Stoff und der zweite in dem zweiten Hydrogelstreifen enthaltene Stoff zur erfindungsgemäßen Verwendung zur Behandlung von Wunden in der Epithelisierungsphase eingesetzt. Hierbei ergeben sich unerwartet die vorstehend genannten vorteilhaften Effekte bei der Wundheilung.

Wie einleitend beschrieben kann der Heilungsprozess unabhängig von der Art der Wunde in unterschiedliche Phasen typisiert werden. Im Fachgebiet wird zwischen Reinigungs- (Entzündungsphase, Inflammation), Granulations- (Proliferation) und Epithelisierungsphase unterschieden. Gemeinsames Merkmal ist, dass unterschiedliche Zelltypen miteinander interagieren, aktiviert werden und proliferieren.

In der Reinigungsphase wird die Blutung üblicherweise durch die Gerinnungskaskade gestoppt. Es bilden sich üblicherweise Gerinnsel aus Fibrinmolekülen, Fibronektin, Vitronectin und Thrombospondin. Sie können als Leitstruktur für einwandernde Zellen dienen. Gleichzeitig können die Thrombozyten im Gerinnsel Wachstumsfaktoren und Zytokine ausschütten und locken damit immunkompetente Zellen an den Ort der Gewebeverletzung. Es kommt gegebenenfalls zu einer lokalen Entzündung, bei der neutrophile Granulozyten eingedrungene Erreger abwehren. Außerdem wird gegebenenfalls abgestorbenes Zellmaterial durch Proteasen beseitigt. In dieser Phase werden meist große Exsudatmengen abgegeben, die die Selbstreinigung der Wunde unterstützen.

Die Granulationsphase ist dadurch gekennzeichnet, dass Monozyten, Endothelzellen und/oder Fibroblasten entlang der provisorischen Fibrinmatrix in das Wundareal einwandern. An den Rändern des frühen Granulationsgewebes können Zellen nekrotisches Gewebe abbauen und am Übergang zum Normalgewebe mit der Neusynthese einer extrazellulären Matrix beginnen. Nach einigen Tagen nimmt die Zellzahl in dieser Matrix massiv zu. Bindegewebe und Blutgefäße werden normalerweise sichtbar. Gegebenenfalls wird neues Kollagen synthetisiert. Während der zweiten Phase werden - sofern erforderlich - neue Gefäße gebildet.

Die Epithelisierungsphase ist, wie vorstehend erwähnt, die dritte Phase der Wundheilung. Sie ist bevorzugt dadurch charakterisiert, dass Epithelzellen in der Wunde erkennbar sind. Bevorzugt überziehen Epithelzellen das noch unfertige Bindegewebe.

Bevorzugt bildet sich, ausgehend vom intakten Epithelgewebe an den Wundrändern, die neue Epidermis. Bevorzugt sorgen ferner Myofibroblasten für eine Kontraktion der Wundränder. Keratinozyten - z.B. vom Wundrand und/oder den Haarwurzelscheiden - können ein neues Epithel bilden.

In einer Ausführungsform ist die Granulationsphase dadurch charakterisiert, dass das Granulationsgewebe wasser- und gefäßärmer wird. Die Kollagenfasern können ausreifen und sich zu Narbengewebe umbilden (Maturation). Es kann dabei gleichzeitig eine Wundkontraktion erfolgen. Bevorzugt haben Fibroblasten ihre Aufbauarbeit beendet und wandeln sich in Fibrozyten und Myofibroblasten um. Die Epithelisierung geht besonders bevorzugt vom Wundrand aus. Hierbei überhäuten bevorzugt die sich neu bildenden Keratinozyten das Granulationsgewebe mit einem zunächst noch sehr feinen Epithel.

Bevorzugt erfolgt die Epithelisierung in einer glatten, feuchten und gut durchbluteten Kriechfläche. Unter Kriechfläche versteht man in diesem Zusammenhang ein ausgebildetes und sauberes Granulationsgewebe, das auf Wundrandniveau reicht. Es sollte im Wesentlichen frei von Nekrosen, Senken, Wundhöhlen oder Hypergranulationen sein. Für die Behandlung einer derartigen Kriechfläche ist das erfindungsgemäße Wundsystem besonders vorteilhaft.

In einer bevorzugten Ausführungsform dauert die Epithelisierungsphase drei Tage bis drei Wochen, bevorzugt fünf Tage bis drei Wochen.

In einer bevorzugten Ausführungsform wird bei der erfindungsgemäßen Verwendung des ersten in dem ersten Hydrogelstreifen enthaltenen Stoffes und des zweiten in dem zweiten Hydrogel enthaltenen Stoffes die Wunde für drei bis fünf Tage befeuchtet. Anschließend kann ein Wechsel des Wundsystems erfolgen.

Weiterhin wird ein Kit als Wundsystem bereitgestellt, umfassend ein erfindungsgemäßes Wundsystem, wobei der erste und/oder der zweite Hydrogelstreifen in einem trockenen Zustand vorliegen, und eine wässrige Lösung zum Aktivieren des Wundsystems.

Das vom Kit umfasste Wundsystem kann hergestellt werden, indem die im trockenen Zustand vorliegenden Hydrogelstreifen, welche bevorzugt auf der Trägerschicht angebracht sind, feuchtigkeitsdicht abgepackt wird. Vorzugsweise liegt das abgepackte Wundsystem in steriler Form vor. Die Sterilisation kann durch eine Gegendruckdampfsterilisation oder durch andere, dem Fachmann als geeignet bekannte Sterilisationsmethoden erfolgen.

Zudem umfasst das Kit eine wässrige Lösung zum Aktivieren des Wundsystems. Bei der wässrigen Lösung handelt es sich bevorzugt um eine wie oben beschriebene salzhaltige, wässrige Lösung. Insbesondere kann es sich um eine Ringerlösung handeln. Durch in Kontakt bringen der ersten und zweiten Hydrogelstreifen in trockenem Zustand mit der wässrigen Lösung werden diese aktiviert; das heißt die Hydrogelstreifen werden in einen feuchten Zustand überführt. In diesem Zustand kann das auf die Wunde aufgebrachte Wundsystem die Wunde feuchthalten und zudem kann ein elektrischer Mikrostrom weiter zur vorteilhaften Wundheilung beitragen.

Das Kit kann weitere optionale Bestandteile, wie beispielsweise eine oder mehrere zusätzliche Druckverteilungsschichten, Klebemittel zum Fixieren des Verbands, Abdichtmittel zur Herstellung einer luftundurchlässigen Abdichtung des Verbandes, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung, enthalten.

Vorstehend wurde gemäß der Erfindung erläutert, dass mittels des erfindungsgemäßen Wundsystems eine vorteilhafte Wundbehandlung erreicht werden kann. Der Erfolg kann alternativ durch weitere Wundsysteme bzw. Wundauflagen erreicht werden, die nachstehend als weitere Aspekte erläutert werden. Grundsätzlich finden für die Aspekte die vorstehend gemachten Erläuterungen zu bevorzugten Ausführungsformen Anwendung.

Ein weiterer Aspekt ist eine Wundauflage, welche ein Substrat und eine Hydrogelschicht umfasst, wobei in der Hydrogelschicht statistisch homogen verteilte Partikel eines ersten und zweiten Stoffes eingearbeitet sind.

Das Substrat kann eine Trägerschicht oder eine Spenderschicht sein.

In einer bevorzugten Ausführungsform ist das Substrat eine Trägerschicht. Die Eigenschaften dieser Trägerschicht entsprechen vorzugsweise den Eigenschaften der oben beschriebenen Trägerschicht.

In einer alternativ bevorzugten Ausführungsform kann das Substrat eine Spenderschicht sein. In einer bevorzugten Ausführungsform umfasst die Spenderschicht einen hydrophilen Polyurethanschaum, der einen Wasseranteil von mindestens 20 Gew.-% Wasser, insbesondere mindestens 30 Gew.-% Wasser und ganz besonders bevorzugt mindestens 35 Gew.-% Wasser, umfasst. Hierbei ist weiterhin bevorzugt vorgesehen, dass der Polyurethanschaum einen Wasseranteil von höchstens 80 Gew.-% Wasser, insbesondere höchstens 70 Gew.-% und ganz besonders bevorzugt von höchstens 65 Gew.-% Wasser, umfasst.

Der Wasseranteil kann vorzugsweise in dem hydrophilen Polyurethanschaum homogen verteilt sein. Insbesondere umfasst der hydrophile Polyurethanschaum dabei einen Wasseranteil von mindestens 10 Gew.-% und höchstens 80 Gew.-% Wasser, wobei das Wasser insbesondere homogen in dem Polyurethanschaum verteilt ist.

Die Menge an Wasser wird wie obenstehend berechnet.

Weiterhin kann der hydrophile Polyurethanschaum einen Retentionswert R von mindestens 20 %, bevorzugt mindestens 30 %, insbesondere mindestens 40 % und ganz besonders bevorzugt von mindestens 50 % aufweisen.

Unabhängig hiervon kann weiterhin bevorzugt vorgesehen sein, dass der hydrophile Polyurethanschaum einen Retentionswert R von höchstens 90 %, insbesondere von höchstens 80 % und ganz besonders von höchstens 70 % aufweist. Der Retentionswert R wird dabei gemäß der folgenden Methode bestimmt.

Der Retentionswert R beschreibt die Menge Wasser, die der Polyurethanschaum in seine Polyurethanmatrix maximal einbinden kann, wobei das Wasser, das in die Poren aufgenommen werden könnte, unberücksichtigt bleibt. Der Retentionswert wird bestimmt, indem ein Probenstück von 5 cm x 5 cm (unter Normklima gelagert) aus einem hydrophilen Polyurethanschaum mit einer Dicke von höchstens 5 mm ausgestanzt und dessen Gewicht unter Normklimabedingungen vermessen wird. Das Probenstück wird hiernach einer freien Absorption mit Wasser analog DIN EN 13726-1 unterworfen. Das Wasser, das von den Poren aufgenommen wurde, wird dem Probenstück mittels einer Rolle (Gewicht 5000 g, Durchmesser 10 cm, Breite 5 cm) herausgequetscht, indem die Probe mehrmals zwischen frische Zellstofftücher gelegt und mit der Rolle überrollt wird. Dieser Vorgang wird solange wiederholt, bis keine Wasserabsorption in den Zellstofftüchern mehr erkennbar ist. Zur Bestimmung des Retentionswertes R wird der Wasseranteil $W_{ww}$, der nach dem Absorbieren und dem Ausquetschen in dem Polyurethanschaum enthalten ist, gemäß DIN EN 14079 gemessen und wie folgt berechnet.

$$R = W_{ww} = \frac{W_{gg} - W_{tt}}{W_{gg}} \bullet 100\% = 52{,}8\,\%\quad\text{(gemessen nach DIN EN 14079)}$$

wobei gilt

$W_{ww}$ = das Gewicht des Wassers, das nach der Absorption und dem Ausquetschen in dem Polyurethanschaum enthalten ist,

$W_{tt}$ = das Gewicht des Probenstücks nach dem Trocknen, und

$W_{gg}$ = das Gewicht des Probenstücks nach Absorption und nach Ausquetschen.

Ganz besonders bevorzugt ist ein hydrophiler Polyurethanschaum, der einen Wasseranteil von mindestens 10 Gew.-% umfasst, wobei der Wasseranteil dem Retentionswert R des Polyurethanschaums entspricht.

Die Wunde sowie gegebenenfalls die Hydrogelschicht können von Anfang an befeuchtet oder mit Feuchtigkeit versorgt werden, wobei die Spenderschicht gleichzeitig durch den Polyurethanschaum eine ausreichende Absorptionskapazität aufweist. Hierdurch werden die Wundheilung eventuell negativ beeinflussende Faktoren der Wundoberfläche entzogen und gleichzeitig Feuchtigkeit und Wasser in einer ausreichenden Menge zur Verfügung gestellt.

Aufgrund der etwas geringeren Absorptionskapazität gegenüber trockenen Polyurethanschäumen, die kein freisetzbares Wasser umfassen und meist sehr schnell sehr viel Wundsekret aufnehmen, wodurch eine trockene Wundoberfläche gebildet wird, ist diese Spenderschicht hervorragend geeignet in der Epithelisierungs- oder Granulationsphase, insbesondere der Epithelisierungsphase der Wundheilung, eingesetzt zu werden. Damit kann der hydrophile Polyurethanschaum in natürlicher Weise die Granulation und/oder die Epithelisierung der Wunde in besonderem Maße fördern.

In einer bevorzugten Ausführungsform kann der hydrophile Polyurethanschaum einen Wasseranteil von mindestens 10 Gew.-% und ein Quellvermögen $\Delta V_1$ von höchstens 80 % aufweisen. Insbesondere weist der hydrophile Polyurethanschaum dabei ein Quellvermögen $\Delta V_1$ von höchstens 60 %, insbesondere von höchstens 40 %, insbesondere von höchstens 30 % und ganz besonders bevorzugt von höchstens 20 % auf. Dabei kann weiterhin vorteilhaft vorgesehen sein, dass der Polyurethanschaum ein Restquellvermögen $\Delta V_1$ von mindestens 5 % aufweist.

Dieses Restquellvermögen kann ausgenützt werden, damit die Wundauflage während der Absorption einen besseren Kontakt zum Wundgrund annehmen kann. Hierbei soll unter dem Quellvermögen $\Delta V_1$ eines Polyurethanschaums die Volumenzunahme verstanden sein, die ein Polyurethanschaum, der seine Absorptionskapazität vollständig erschöpft hat, im Vergleich zu einem Polymerurethanschaum mit einem Wassergehalt von mindestens 10 Gew.-% Wasser erfährt. Das Quellvermögen soll dabei gemäß einem hierin beschriebenen Test ermittelt werden.

Das Quellvermögen $\Delta V_1$ eines Polyurethanschaums beschreibt die Volumenänderung, die ein wasserhaltiger Polyurethanschaum erfährt, nachdem er seine maximale Absorption erreicht hat. Zur Bestimmung des Quellvermögens $\Delta V_1$ werden die räumlichen Abmessungen eines Probenstücks des wasserhaltigen Polymerschaums und die räumlichen Abmessungen dieses Probenstücks nach vollständiger Absorption gemäß der freien Absorption nach DIN EN 13726-1 bestimmt. Zur Bestimmung der Dicke (Höhe) wird ein Dickenmessgerät mit 25 cm$^2$ Teller verwendet, wobei eine Belastung von 2 g/cm$^2$ gemäß EN ISO 9073-2 eingestellt wird. Die laterale Ausdehnung (Länge, Breite) wird mittels einer Schieblehre bestimmt, ohne dass das Probenstück deformiert wird. Zur Bestimmung der Ausdehnung wird das jeweilige Probenstück spannungsfrei auf eine glatte Oberfläche abgelegt. Die Volumenänderung nach Absorption entspricht dem Quellvermögen $\Delta V_1$ des wasserhaltigen Polyurethanschaums, wobei alle drei Raumrichtungen beachtet werden.

$$\Delta V_1 = \frac{V_2 - V_1}{V_1} \bullet 100\% = \frac{(l_2 \bullet b_2 \bullet h_2) - (l_1 \bullet b_1 \bullet h_1)}{(l_1 \bullet b_1 \bullet h_1)} \bullet 100\%$$

wobei gilt

$V_1$ = das Volumen des wasserhaltigen Probenstücks und

$V_2$ = das Volumen des Probenstücks nach vollständiger Absorption.

Als Polyurethanschaum kann jeder heute in der modernen Wundbehandlung übliche hydrophile Polyurethanschaum verwendet werden, der einen Wasseranteil in seine Polyurethanmatrix aufnimmt und eine ausreichende Absorption aufweist. Damit ist unter einem hydrophilen Polyurethanschaum ein solcher Polyurethanschaum zu verstehen, der eine Flüssigkeit in seine Polyurethanmatrix und in seine Poren aufnehmen und speichern, somit absorbieren, und zumindest einen Teil der aufgenommenen Flüssigkeit wieder abgeben kann. Als hydrophile Polymerschäume sind hierbei insbesondere offenporige, hydrophile Polyurethanschäume geeignet. Demgemäß umfasst eine besonders bevorzugte Wundauflage eine zweite Schicht, die einen offenporigen, hydrophilen Polyurethanschaum umfasst.

**EP 3 184 125 B1**

Bevorzugt sollen solche Polyurethanschäume eingesetzt werden, die eine hohe Absorptionskapazität aufweisen. Diese Absorptionskapazität soll vorhanden sein, obwohl der Polyurethanschaum einen Anteil seines Eigengewichts an Wasser in seine Polymermatrix aufgenommen hat. Bevorzugt ist ein Polyurethanschaum, der einen Wasseranteil von mindestens 10 Gew.-% und höchstens 80 Gew.-% umfasst und der eine freie Absorption $A_2$ von mindestens 10 g/g, insbesondere mindestens 12 g/g und ganz besonders bevorzugt von mindestens 15 g/g aufweist, wobei die freie Absorption $A_2$ gemäß der DIN-EN 13726-1 (2002) bestimmt wird. Hierbei ist die freie Absorption $A_2$ die freie Absorption des Wasser-enthaltenden Polyurethanschaums.

Weiterhin bevorzugt kann der hydrophile Polyurethanschaum eine durchschnittliche Porengröße von weniger als 1000 $\mu$m, insbesondere 100 $\mu$m bis 1000 $\mu$m, insbesondere 100 $\mu$m bis 500 $\mu$m und ganz besonders bevorzugt 100 $\mu$m bis 300 $\mu$m, aufweisen. Hierbei kann insbesondere vorgesehen sein, dass die durchschnittliche Porengröße an der ersten Seite der zweiten Schicht gleich groß ist wie die Porengröße im Inneren der zweiten Schicht und/oder gleich groß ist wie an der zweiten Seite der zweiten Schicht. Weiterhin bevorzugte hydrophile Polyurethanschäume weisen eine Dichte von weniger als 150 kg/m$^3$, insbesondere weniger als 140 kg/m$^3$ und ganz besonders bevorzugt 50 kg/m$^3$ bis 120 kg/m$^3$ auf. Die Bestimmung der Porengröße erfolgt bevorzugt mikroskopisch, wobei ein Probenquerschnitt mikroskopiert wurde; die angegebene Porengröße entspricht dem Mittelwert von fünf zufällig ausgewählten und ausgemessenen Poren pro Probe.

Als besonders vorteilhafte Ausführungsform hat sich gezeigt, dass die Spenderschicht eine Dicke von 0,1 bis 5,0 mm, insbesondere von 0,5 bis 5,0 mm und ganz besonders bevorzugt von 0,5 bis 3,0 mm aufweist. Solche Schichtdicken zeigen einerseits die Fähigkeit auf, ein von einer Wunde abgegebenes Wundexsudat aufzunehmen und gleichzeitig eine ausreichende Menge an Wasser oder Feuchtigkeit einer Wunde bereitstellen zu können. Diese Schichtdicken können an jeder Stelle gleich sein oder in verschiedenen Bereichen verschiedene Werte annehmen. Insbesondere ist auch vorgesehen, dass die Spenderschicht abgeflachte Ränder aufweist.

Die Hydrogelschicht ist aus einem Hydrogel beziehungsweise einer Hydrogelmatrix wie oben beschrieben aufgebaut. Die Hydrogelschicht enthält statistisch homogen verteilte Partikel des ersten und zweiten Stoffes. Für den ersten und zweiten Stoff gilt das oben Beschriebene. Eine statistisch homogene Verteilung der Partikel des ersten und zweiten Stoffes kann bedeuten, dass jede gleich große Volumeneinheit bevorzugt gleich viele Partikel des ersten und zweiten Stoffes enthält.

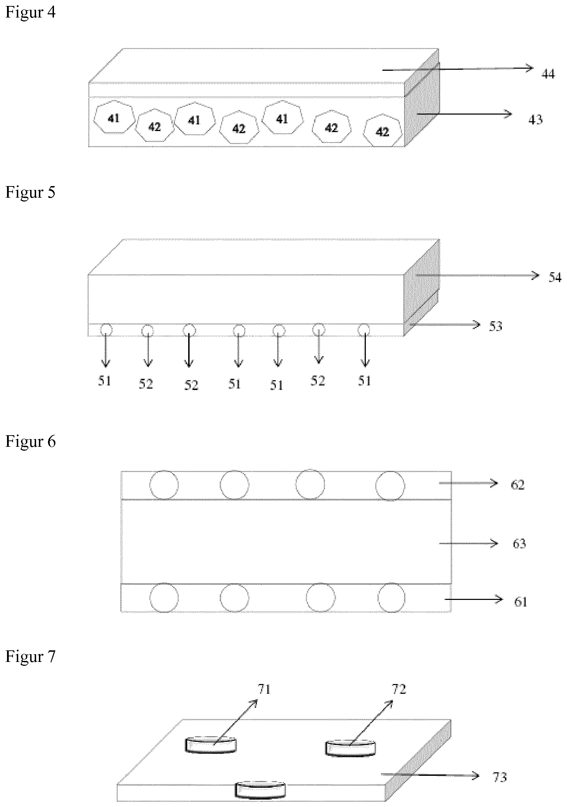

Die Hydrogelschicht kann bevorzugt flächig auf das Substrat aufgebracht sein. In einer bevorzugten Ausführungsform kann zwischen Substrat und Hydrogelschicht eine Adhäsivschicht vorliegen, um eine haltbare Verbindung zwischen Substrat und Hydrogelschicht bereitzustellen. In einer bevorzugten Ausführungsform ist die Hydrogelschicht, welche die statistisch homogen verteilten Partikel des ersten und zweiten Stoffes enthält, die Wundkontaktschicht, d.h. die direkt mit der Wunde in Kontakt stehende Schicht. Eine bevorzugte Ausführungsform für diesen Aspekt kann durch Figur 4 (nicht Erfindungsgemäß) veranschaulicht werden.

In Figur 4 bedeuten

41 erster Stoff
42 zweiter Stoff
43 Hydrogelschicht
44 Substrat

Ein weiterer Aspekt ist eine Wundauflage, welche eine Trägerschicht und eine Hydrogelschicht umfasst, wobei in der Trägerschicht verteilte Partikel eines ersten und zweiten Stoffes eingearbeitet sind.

Die Trägerschicht ist wie oben beschreiben aufgebaut. Die Trägerschicht enthält Partikel des ersten und zweiten Stoffes, wobei diese Partikel bevorzugt statistisch homogen verteilt in der Trägerschicht vorliegen. Für den ersten und zweiten Stoff gilt das oben Beschriebene. Eine statistisch homogene Verteilung der Partikel des ersten und zweiten Stoffes kann bedeuten, dass jede gleich große Volumeneinheit bevorzugt gleich viele Partikel des ersten und zweiten Stoffes enthält.

Die Hydrogelschicht ist aus einem Hydrogel beziehungsweise einer Hydrogelmatrix wie oben beschrieben aufgebaut.

Die Hydrogelschicht kann bevorzugt flächig auf die Trägerschicht aufgebracht sein. In einer bevorzugten Ausführungsform kann zwischen Trägerschicht und Hydrogelschicht eine Adhäsivschicht liegen, um eine haltbare Verbindung zwischen Substrat und Hydrogelschicht bereitzustellen. In einer bevorzugten Ausführungsform ist die Hydrogelschicht die Wundkontaktschicht, d.h. die direkt mit der Wunde in Kontakt stehende Schicht. Eine bevorzugte Ausführungsform für diesen Aspekt kann durch Figur 5 (nicht Erfindungsgemäß) veranschaulicht werden.

In Figur 5 bedeuten

51 erster Stoff
52 zweiter Stoff

53 Hydrogelschicht
54 Trägerschicht

Ein weiterer Aspekt ist eine Wundauflage, welche eine erste Trägerschicht, welche einen ersten Stoff enthält, eine zweite Trägerschicht, welche einen zweiten Stoff enthält, und eine Hydrogelschicht umfasst.

Die erste Trägerschicht ist wie die oben stehende Trägerschicht aufgebaut, wobei die erste Trägerschicht Partikel eines ersten Stoffes enthält. Die Partikel des ersten Stoffes liegen in der ersten Trägerschicht verteilt vor, vorzugsweise statistisch homogen verteilt.

Die zweite Trägerschicht ist wie die oben stehende Trägerschicht aufgebaut, wobei die zweite Trägerschicht Partikel eines zweiten Stoffes enthält. Die Partikel des zweiten Stoffes liegen in der zweiten Trägerschicht verteilt vor, vorzugsweise statistisch homogen verteilt.

Eine statistisch homogene Verteilung der Partikel des ersten und zweiten Stoffes kann bedeuten, dass jede gleich große Volumeneinheit bevorzugt gleich viele Partikel des ersten und zweiten Stoffes enthält.

Die Hydrogelschicht ist aus einem Hydrogel beziehungsweise einer Hydrogelmatrix wie oben beschrieben aufgebaut.

Die Hydrogelschicht kann bevorzugt flächig zwischen der ersten und der zweiten Trägerschicht angebracht sein. In einer bevorzugten Ausführungsform kann zwischen der ersten Trägerschicht und der Hydrogelschicht und/oder zwischen der zweiten Trägerschicht und der Hydrogelschicht eine Adhäsivschicht liegen, um eine haltbare Verbindung zwischen Substrat und Hydrogelschicht bereitzustellen. Eine bevorzugte Ausführungsform für diesen Aspekt kann durch Figur 6 (nicht Erfindungsgemäß) veranschaulicht werden.

In Figur 6 bedeuten

61 erste Trägerschicht enthaltend ersten Stoff
62 zweite Trägerschicht enthaltend zweiten Stoff
63 Hydrogelschicht

Ein weiterer Aspekt ist eine Wundauflage, welche eine Hydrogelschicht umfasst, welche in Abständen definierte Partikel enthält. Hierbei gibt es zwei unterschiedliche Typen von Partikel. Der erste "Partikeltyp" umfasst einen ersten Stoff und der zweite Partikeltyp umfass einen zweiten Stoff.

Die Hydrogelschicht ist aus einem Hydrogel beziehungsweise einer Hydrogelmatrix wie oben beschrieben aufgebaut. Die definierten Partikel aus dem ersten wie aus dem zweiten Stoff können als erste und zweite Elektrodenpunkte betrachtet werden. Diese ersten und zweiten Elektrodenpunkte können vorzugsweise durch Stanzen in die Hydrogelschicht eingebracht werden. Auf diese Weise liegen die ersten und zweiten Elektrodenpunkte zwar bevorzugt unter der einen Oberfläche der Hydrogelschicht, sind vorzugsweise jedoch nicht vollständig vom Hydrogel umschlossen. Die definierten ersten und zweiten Elektrodenpunkte sind bevorzugt so angeordnet, dass sich weder zwei erste oder zweite Elektrodenpunkte noch ein erster und ein zweiter Elektrodenpunkt berühren. Die Hydrogelschicht wird vor der Verwendung in der Wundbehandlung durch Tränken der obenstehend beschriebenen salzhaltigen Lösung aktiviert, welche dann zusammen mit dem Wundexsudat eine Elektrolytlösung bilden, sodass zwischen den ersten und zweiten Elektrodenpunkten ein elektrischer Mikrostrom fließen kann. Die Hydrogelschicht wird vorzugsweise mit der Seite auf der Wunde aufgebracht, in der die ersten und zweiten Elektrodenpunkte aufgebracht sind. Eine bevorzugte Ausführungsform für diesen Aspekt kann durch Figur 7 (nicht Erfindungsgemäß) veranschaulicht werden.

In Figur 7 bedeuten

71 erster Elektrodenpunkt
72 zweiter Elektrodenpunkt
73 Hydrogelschicht

Ein weiterer Aspekt ist eine Wundauflage, welche eine Hydrogelschicht umfasst, wobei auf der einen Oberfläche der Hydrogelschicht in Abständen definierte Partikel (Elektrodenpunkte) einmal aus einem ersten Stoff und einmal aus einem zweiten Stoff aufgebracht sind.

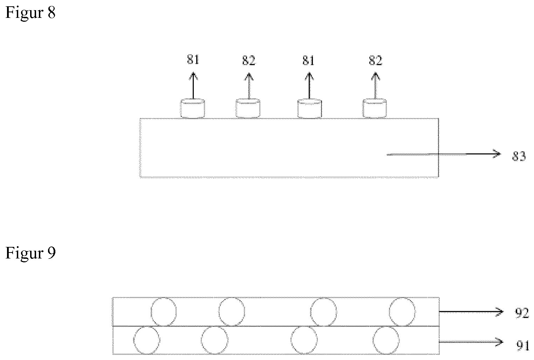

Die Hydrogelschicht ist aus einem Hydrogel beziehungsweise einer Hydrogelmatrix wie oben beschrieben aufgebaut. Die definierten Partikel aus dem ersten wie aus dem zweiten Stoff können als erste und zweite Elektrodenpunkte betrachtet werden. Diese ersten und zweiten Elektrodenpunkte können vorzugsweise eine Noppenform aufweisen. Zum Aufbringen der ersten und zweiten Elektrodenpunkte kann vorzugsweise eine Maske, bevorzugt Halbmasken verwendet werden. Die Maske, bevorzugt die zwei Halbmasken, wird/werden über die Hydrogelschicht gelegt und durch die in der Maske vorhandenen Aussparungen können der erste und der zweite Stoff auf die Hydrogelschicht aufgebracht werden. Auf diese Weise liegen sowohl die ersten als auch die zweiten Elektrodenpunkte auf einer Hydrogelschicht vor, sind vorzugsweise jedoch nur mit ihren Grundflächen mit dem Hydrogel in Kontakt. Die definierten ersten und zweiten Elektrodenpunkte sind bevorzugt so angeordnet, dass sich weder zwei erste oder zweite Elektrodenpunkte noch ein erster und ein zweiter Elektrodenpunkt berühren. Die Hydrogelschicht wird vor der Verwendung in der Wundbehandlung durch Tränken der obenstehend beschriebenen salzhaltigen Lösung aktiviert, welche dann zusammen mit dem Wundexsudat eine Elektrolytlösung bildet, sodass zwischen den ersten und zweiten Elektrodenpunkten ein elektrischer Mikrostrom fließen kann. Die Hydrogelschicht wird vorzugsweise mit der Seite auf der Wunde aufgebracht, auf der die ersten und zweiten Elektrodenpunkte aufgebracht sind. Eine bevorzugte Ausführungsform für diesen Aspekt kann durch Figur 8 (nicht Erfindungsgemäß) veranschaulicht werden.

In Figur 8 bedeuten

81 erster Elektrodenpunkt
82 zweiter Elektrodenpunkt
83 Hydrogelschicht

Ein weiterer Aspekt ist ein Wundversorgungssystem, welches eine Hydrogelschicht und eine Auflageschicht umfasst, wobei die Hydrogelschicht Partikel aus einem ersten Stoff enthält und die Auflageschicht einen zweiten Stoff umfasst.

Die Hydrogelschicht ist aus einem Hydrogel beziehungsweise einer Hydrogelmatrix wie oben beschrieben aufgebaut. Die in der Hydrogelschicht enthaltenen Partikel aus einem ersten Stoff sind vorzugsweise Zinkpartikel. Die Partikel können vorzugsweise vollständig von der Hydrogelschicht umgeben sein, so dass sie weder an noch oberhalb einer Oberfläche der Hydrogelschicht sind. In einer bevorzugten Ausführungsform kann das die Partikel aus dem ersten Stoff enthaltende Hydrogel mit einer Spritze auf die Wunde aufgebracht und anschließend gegebenenfalls unter Verwendung geeigneter Utensilien, wie einem Spatel, zu einer Schicht geformt werden. Diese Schicht kann als Wundkontaktschicht betrachtet werden.

Bei der Auflageschicht kann es sich vorzugsweise um eine Silberwundauflage, wie z.B. Atrauman Ag®, handeln. Diese Auflageschicht wird vorzugsweise zur Überdeckung der Hydrogelschicht verwendet.

Die Hydrogelschicht kann vor der Verwendung in der Wundbehandlung durch Tränken der obenstehend beschriebenen salzhaltigen Lösung aktiviert werden, welche dann zusammen mit dem Wundexsudat eine Elektrolytlösung bildet, sodass ein elektrischer Mikrostrom fließen kann. Eine bevorzugte Ausführungsform für diesen Aspekt kann durch Figur 9 (nicht Erfindungsgemäß) veranschaulicht werden.

In Figur 9 bedeuten

91 Hydrogelschicht, welche einen ersten Stoff enthält
92 Auflageschicht, welche einen zweiten Stoff enthält

Die Erfindung kann durch folgende Beispiele veranschaulicht werden:
Beispiel 1: Zusammensetzung für Hydrogelstreifen
Erster Hydrogelstreifen:

| | |
|---|---|
| NCO-terminiertes EO-PEO-Präpolymer (Aquapol®) | 12,5 Gew.-% |
| $NH_2$-terminiertes EO-PEO- Präpolymer (Jeffamine®) | 8,66 Gew.-% |
| Wasser | 59,38 Gew.-% |
| Propylenglykol | 16,5 Gew.-% |
| NaCl | 0,96 Gew.-% |
| Zink (Zn) | 2,00 Gew.-% |

Zweiter Hydrogelstreifen:

| | |
|---|---|
| NCO-terminiertes EO-PEO-Präpolymer (Aquapol®) | 12,5 Gew.-% |
| $NH_2$-terminiertes EO-PEO- Präpolymer (Jeffamine®) | 8,66 Gew.-% |
| Wasser | 59,38 Gew.-% |
| Propylenglykol | 16,5 Gew.-% |
| NaCl | 0,96 Gew.-% |
| Silberoxid ($Ag_2O$) | 2,00 Gew.-% |

Beispiel 2 Zusammensetzung für Hydrogelstreifen

Erster Hydrogelstreifen:

| | |
|---|---|
| NCO-terminiertes EO-PEO-Präpolymer (Aquapol®) | 12,5 Gew.-% |
| $NH_2$-terminiertes EO-PEO- Präpolymer (Jeffamine®) | 8,66 Gew.-% |
| Wasser | 60,88 Gew.-% |
| Propylenglykol | 16,5 Gew.-% |
| NaCl | 0,96 Gew.-% |
| Zink (Zn) | 0,50 Gew.-% |

Zweiter Hydrogelstreifen:

| | |
|---|---|
| NCO-terminiertes EO-PEO-Präpolymer (Aquapol®) | 12,5 Gew.-% |
| $NH_2$-terminiertes EO-PEO- Präpolymer (Jeffamine®) | 8,66 Gew.-% |
| Wasser | 60,88 Gew.-% |
| Propylenglykol | 16,5 Gew.-% |
| NaCl | 0,96 Gew.-% |
| Silberoxid ($Ag_2O$) | 0,50 Gew.-% |

**Patentansprüche**

1. Wundsystem umfassend:

   (a) Trägerschicht,
   (b) mindestens zwei Hydrogelstreifen

   wobei ein erster Hydrogelstreifen einen ersten Stoff und ein zweiter Hydrogelstreifen einen zweiten Stoff enthält und wobei der erste und der zweite Stoff ein unterschiedliches Standardpotential $E°$ aufweisen, gemessen bei 25°C; 101,3 kPa, pH=0, Ionenaktivität = 1, und wobei der erste und/oder der zweite Hydrogelstreifen eine Hydrogelmatrix umfasst,
   wobei die Hydrogelmatrix ein Polyurethan-Polyharnstoff-Copolymer umfasst, und
   wobei die Hydrogelstreifen die Wundkontaktschicht sind, und
   wobei die Hydrogelstreifen der Wunde zugewandt sind und im direkten Kontakt mit der Wunde stehen, und
   wobei die Trägerschicht einen Polymerschaum umfasst.

2. Wundsystem nach Anspruch 1, wobei die Trägerschicht einen Film, einen Schaum, ein Gewebe, ein Gestrick, ein Filament, einen Vliesstoff, verwobene Materialien und/oder Kombinationen daraus umfasst.

3. Wundsystem nach einem der vorstehenden Ansprüche, wobei der erste und der zweite Hydrogelstreifen das gleiche Hydrogel oder die gleiche Hydrogelmatrix umfassen.

4. Wundsystem nach einem der vorstehenden Ansprüche, wobei der erste Stoff und der zweite Stoff jeweils ein Metall und/oder ein entsprechendes Metallsalz sind.

5. Wundsystem nach einem der vorstehenden Ansprüche, wobei der erste Stoff Zink ist.

6. Wundsystem nach einem der vorstehenden Ansprüche, wobei der zweite Stoff Silber und/oder ein Silbersalz ist.

7. Wundsystem nach einem der vorstehenden Ansprüche, wobei der erste und/oder der zweite Hydrogelstreifen ein zusätzliches Salz umfasst.

8. Erster und zweiter Stoff zur Verwendung in der therapeutischen Behandlung von Wunden am menschlichen oder tierischen Körper, wobei der erste Stoff in einem ersten Hydrogelstreifen und der zweite Stoff in einem zweiten Hydrogelstreifen enthalten sind, wobei die Hydrogelstreifen auf eine Trägerschicht aufgebracht sind, und wobei der erste und der zweite Stoff ein unterschiedliches Standardpotential $E°$ aufweisen, gemessen bei 25°C; 101,3 kPa, pH=0, Ionenaktivität =1, und wobei der erste und/oder der zweite Hydrogelstreifen eine Hydrogelmatrix umfasst,

wobei die Hydrogelmatrix ein Polyurethan-Polyharnstoff-Copolymer umfasst, und
wobei die Hydrogelstreifen die Wundkontaktschicht sind, und
wobei die Hydrogelstreifen der Wunde zugewandt sind und im direkten Kontakt mit der Wunde stehen, und
wobei die Trägerschicht einen Polymerschaum umfasst.

9. Erster und zweiter Stoff zur Verwendung nach Anspruch 8, wobei der erste und zweite Stoff zur Behandlung von Wunden in der Epithelisierungsphase eingesetzt wird.

10. Erster und zweiter Stoff zur Verwendung nach Anspruch 8 oder 9, wobei die Wunde für drei bis fünf Tage befeuchtet wird.

**Claims**

1. Wound system comprising:

   (a) a backing layer,
   (b) at least two hydrogel strips

   wherein a first hydrogel strip contains a first substance and a second hydrogel strip contains a second substance and wherein the first and the second substances have a different standard potential $E°$, measured at 25°C; 101.3 kPa, pH=0, ionic activity = 1, and wherein the first and/or the second hydrogel strip comprises a hydrogel matrix, wherein the hydrogel matrix comprises a polyurethane-polyurea copolymer, and wherein the hydrogel strips are the wound contact layer, and
   wherein the hydrogel strips face the wound and are in direct contact with the wound, and
   wherein the backing layer comprises a polymer foam.

2. Wound system according to claim 1, wherein the backing layer comprises a film, a foam, a fabric, a knitted fabric, a filament, a non-woven fabric, woven materials and/or combinations thereof.

3. Wound system according to any one of the preceding claims, wherein the first and second hydrogel strips comprise the same hydrogel or hydrogel matrix.

4. Wound system according to any one of the preceding claims, wherein the first substance and the second substance are each a metal and/or a corresponding metal salt.

5. Wound system according to any one of the preceding claims, wherein the first substance is zinc.

6. Wound system according to any one of the preceding claims, wherein the second substance is silver and/or a silver salt.

7. Wound system according to any one of the preceding claims, wherein the first and/or second hydrogel strip comprises an additional salt.

8. First and second substances for use in the therapeutic treatment of wounds on the human or animal body, wherein the first substance is contained in a first hydrogel strip and the second substance is contained in a second hydrogel strip, wherein the hydrogel strips are applied to a backing layer, and wherein the first and the second substance have a different standard potential $E°$, measured at 25°C; 101.3 kPa, pH=0, ionic activity =1, and wherein said first and/or second hydrogel strips comprise a hydrogel matrix, said hydrogel matrix comprising a polyurethane-polyurea copolymer, and
   wherein the hydrogel strips are the wound contact layer, and
   wherein the hydrogel strips face the wound and are in direct contact with the wound, and
   wherein the backing layer comprises a polymer foam.

9. First and second substances for use according to claim 8, wherein the first and second substances are used for the treatment of wounds in the epithelization phase.

10. First and second substances for use according to claim 8 or 9, wherein the wound is moistened for three to five days.

**Revendications**

1. Système de plaies comprenant:

   a) une couche de support,
   b) au moins deux bandes d'hydrogel

   une première bande d'hydrogel contenant une première substance et une deuxième bande d'hydrogel contenant une deuxième substance et la première et la deuxième substance ayant un potentiel standard $E°$ différent, mesuré à 25°C ; 101,3 kPa, pH=0, activité ionique = 1, et la première et/ou la deuxième bande d'hydrogel comprenant une matrice d'hydrogel,
   la matrice d'hydrogel comprenant un copolymère polyuréthane-polyurée, et les bandes d'hydrogel constituant la couche de contact de la plaie, et
   les bandes d'hydrogel faisant face à la plaie et étant en contact direct avec la plaie, et
   la couche de support comprenant une mousse polymère.

2. Système de plaie selon la revendication 1, la couche de support comprenant un film, une mousse, un tissu, un tricot, un filament, un non-tissé, des matériaux tissés et/ou des combinaisons de ceux-ci.

3. Système de plaies selon l'une des revendications précédentes, les première et deuxième bandes d'hydrogel comprenant le même hydrogel ou la même matrice d'hydrogel.

4. Système de plaies selon l'une des revendications précédentes, la première substance et la deuxième substance étant chacun un métal et/ou un sel métallique correspondant.

5. Système de plaies selon l'une des revendications précédentes, la première substance étant du zinc.

6. Système de plaie selon l'une des revendications précédentes, la deuxième substance étant de l'argent et/ou un sel d'argent.

7. Système de plaies selon l'une des revendications précédentes, la première et/ou la deuxième bande d'hydrogel comprenant un sel supplémentaire.

8. Une première et une deuxième substance pour utilisation dans le traitement thérapeutique de plaies sur le corps humain ou animal, la première substance étant contenue dans une première bande d'hydrogel et la deuxième substance étant contenue dans une deuxième bande d'hydrogel, les bandes d'hydrogel étant appliquées sur une couche de support, et la première et la deuxième substance ayant un potentiel standard $E°$ différent, mesuré à 25°C ; 101,3 kPa, pH = 0, activité ionique = 1, et la première et/ou la deuxième bande d'hydrogel comprenant une matrice d'hydrogel, la matrice d'hydrogel comprenant un copolymère polyuréthane-polyurée, et
   les bandes d'hydrogel constituant la couche de contact de la plaie, et
   les bandes d'hydrogel faisant face à la plaie et étant en contact direct avec la plaie, et
   la couche de support comprenant une mousse polymère.

9. Première et deuxième substances pour utilisation selon la revendication 8, la première et la deuxième substances étant utilisées pour le traitement des plaies dans la phase d'épithélialisation.

10. Première et deuxième substances pour utilisation selon la revendication 8 ou 9, la plaie étant humidifiée pendant trois à cinq jours.

Figur 1

Figur 2

Figur 3

Figur 4

Figur 5

Figur 6

Figur 7

Figur 8

Figur 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010000451 A
- WO 2011141454 A
- WO 2014178945 A